# EUROPEAN PATENT SPECIFICATION

(11) **EP 4 186 948 B1**
(45) Date of publication and mention of the grant of the patent: **16.09.2026**
(21) Application number: 21846781.9
(22) Date of filing: 15.07.2021
(51) Int. Cl.: C08L 101/14, A61F 13/15, A61F 13/53, B01J 20/26

(54) **WATER-ABSORBENT RESIN COMPOSITION, METHOD FOR PRODUCING WATER-ABSORBENT RESIN COMPOSITION, AND METHOD FOR SLOWING WATER ABSORPTION RATE OF WATER-ABSORBENT RESIN PARTICLES**
WASSERABSORBIERENDE HARZZUSAMMENSETZUNG, VERFAHREN ZUR HERSTELLUNG EINER WASSERABSORBIERENDEN HARZZUSAMMENSETZUNG UND VERFAHREN ZUR VERZÖGERUNG DER WASSERABSORPTIONSRATE WASSERABSORBIERENDER HARZPARTIKEL
COMPOSITION DE RÉSINE ABSORBANT L'EAU, PROCÉDÉ DE PRODUCTION DE COMPOSITION DE RÉSINE ABSORBANT L'EAU ET PROCÉDÉ DE RALENTISSEMENT DE LA VITESSE D'ABSORPTION D'EAU DE PARTICULES DE RÉSINE ABSORBANT L'EAU

(30) Priority: 22.07.2020 JP 2020125174
(43) Date of publication of application: 31.05.2023
(73) Proprietor: Sumitomo Seika Chemicals Co., Ltd., Kako-gun, Hyogo 675-0145 (JP)
(72) Inventor: MURAKAMI, Masahiro, Himeji-shi, Hyogo 672-8076 (JP)
(74) Representative: Mewburn Ellis LLP
(86) International application number: PCT/JP2021/026699
(87) International publication number: WO 2022/019218

(56) References cited:
- EP-A1- 3 677 628
- WO-A1-2017/170604
- JP-A- 2001 098 170
- JP-A- 2005 029 751
- JP-A- 2005 186 016
- JP-A- 2005 509 696
- JP-A- 2014 094 379
- JP-A- 2014 204 799
- JP-A- 2015 009 042
- JP-A- S6 228 402
- JP-A- S6 433 158
- US-A1- 2020 122 117

## Description

The present invention relates to a water-absorbent resin composition, a method for producing the water-absorbent resin composition, and a method for reducing an absorption rate of water-absorbent resin particles, and more particularly relates to a water-absorbent resin composition constituting an absorber suitably used for sanitary materials such as disposable diapers, sanitary napkins, and incontinence pads, a method for producing the water-absorbent resin composition, and a method for reducing the absorption rate of water-absorbent resin particles.

### BACKGROUND ART

In recent years, water-absorbent resins have been widely used in a field of sanitary materials such as disposable diapers, sanitary napkins, and incontinence pads.

As such water-absorbent resins, a partially neutralized acrylic acid polymer crosslinked product is considered to be a preferable water-absorbent resin because it has an excellent water-absorbent ability, and the acrylic acid as a raw material thereof is easily industrially available, so that it can be produced at a low cost with a constant quality. Further reasons for such preference include its advantages such as its lower possibility of causing decay or deterioration (see, for example, JP H3-227301).

On the other hand, absorbent articles such as disposable diapers, sanitary napkins, or incontinence pads mainly include an absorber that absorbs and holds body fluids such as urine and menstrual blood excreted from a body and is disposed in a central portion, a liquid-permeable top surface sheet (top sheet) disposed on a side in contact with the body, and a liquid-impermeable back surface sheet (back sheet) disposed on an opposite side in contact with the body. **In** addition, the absorber is usually composed of hydrophilic fibers such as pulp and water-absorbent resins. JP 2005-186016 A concerns an absorbent containing a water absorbent resin, water insoluble particles and an oxycarboxylic acid. EP 3677628 A1 concerns a superabsorbent polymer composition including: superabsorbent polymer particles including a crosslinked polymer of water-soluble ethylenically unsaturated monomers including acid groups, of which a least a part are neutralized, and an antimicrobial agent having a controlled particle size, containing a chelating agent including EDTA or an alkali metal salt thereof; a mixture of an organic acid and a silicate-based salt; and an agent for controlling a particle size. US 2020/0122117 A1 concerns a superabsorbent polymer composition including superabsorbent polymer particles, a chelating agent and a mixture of an organic acid and a silicate-based salt.

### SUMMARY OF THE INVENTION

### PROBLEMS TO BE SOLVED BY THE INVENTION

In such absorbent articles, the absorption rate of the water-absorbent resin particles contained in the absorber is required to be high. However, if the absorption rate is too high, when liquid is introduced into the absorber, the water-absorbent resin particles quickly absorb the liquid at the place where the liquid is introduced, and the liquid is less likely to spread over the entire absorber, so that a wide range of the absorber may not be effectively utilized. In such a case, there is a problem that the liquid introduced into the absorber for a plurality of times stagnates around the water-absorbent resin particles around the introduced portion and locally reaches saturation, and the liquid that has not been absorbed causes a re-wet phenomenon (that is, a phenomenon that the liquid reverses from the absorber and it is felt as wet when touched with a hand).

As a method of diffusing the liquid over the absorber to suppress re-wet of the absorber, there is a method of using water-absorbent resin particles with a reduced absorption rate. Furthermore, as a method for reducing the absorption rate, there is a method of increasing the particle size of the water-absorbent resin particles to reduce a surface area of the water-absorbent resin particles. However, when the particle size of the water-absorbent resin particles increases, a grittiness due to large particles increases, which causes a problem that feel of the absorber is deteriorated.

Under such circumstances, a main object of the present invention is to provide a water-absorbent resin composition in which the absorption rate is reduced without increasing the particle size.

### MEANS FOR SOLVING THE PROBLEM

The present inventors have conducted intensive studies in order to solve the above problems. As a result, the present inventors have found that a water-absorbent resin composition containing water-absorbent resin particles with a median particle size of 300 to 450 µm and an acidic compound with a median particle size of 100 to 300 µm has a low absorption rate although the water-absorbent resin composition has a particle diameter of 450 µm or less, which is not large. The present invention has been completed through further intensive studies based on such findings.

In other words, the present invention provides an invention with the following configuration.
In a first aspect, the invention provides a water-absorbent resin composition including: water-absorbent resin particles with a median particle size of 300 to 450 µm; and an acidic compound with a median particle size of 100 to 300 µm, wherein an absorption rate A of the water-absorbent resin particles is 10 to 40 seconds.
In some embodiments, a difference (B - A) between an absorption rate A of the water-absorbent resin particles and an absorption rate B of the water-absorbent resin composition is 1 second or more.
In some embodiments, the absorption rate B of the water-absorbent resin composition is 4 to 130 seconds.
In some embodiments, a first acid dissociation constant of the acidic compound is 0.1 to 5.0.
In a second aspect, the invention provides an absorber including: water-absorbent resin particles with a median particle size of 300 to 450 µm; and an acidic compound with a median particle size of 100 to 300 µm, wherein an absorption rate A of the water-absorbent resin particles is 10 to 40 seconds, and wherein the water-absorbent resin particles and the acidic compound are mixed.
In a third aspect, the invention provides an absorbent article including the absorber according to the second aspect.
In a fourth aspect, the invention provides a method for producing a water-absorbent resin composition, the method including a step of mixing water-absorbent resin particles with a median particle size of 300 to 450 µm and an acidic compound with a median particle size of 100 to 300 µm, wherein an absorption rate A of the water-absorbent resin particles is 10 to 40 seconds.
In some embodiments, a temperature in the mixing step is 0 to 90°C and the relative humidity is 30 to 75%.
In some embodiments, an amount of the acidic compound is 0.05 to 30 parts by mass relative to 100 parts by mass of the water-absorbent resin particles.
In some embodiments, a ratio (T/S) of a median particle size T (µm) of the water-absorbent resin particles to a median particle size S (µm) of the acidic compound is 0.1 to 30.
In a fifth aspect, the invention provides a method for reducing an absorption rate of water-absorbent resin particles, the method including a step of mixing an acidic compound with a median particle size of 100 to 300 µm and water-absorbent resin particles with a median particle size of 300 to 450 µm, wherein an absorption rate A of the water-absorbent resin particles is 10 to 40 seconds.

### ADVANTAGES OF THE INVENTION

According to the present invention, it is possible to provide a water-absorbent resin composition in which the absorption rate is reduced without increasing the particle size. Furthermore, the present invention can also provide a method for producing the water-absorbent resin composition and a method for reducing the absorption rate of water-absorbent resin particles.

### EMBODIMENTS OF THE INVENTION

### 1. Water-absorbent resin composition

The water-absorbent resin composition of the present invention contains water-absorbent resin particles with a median particle size of 300 to 450 µm and an acidic compound with a median particle size of 100 to 300 µm. The water-absorbent resin composition of the present invention with such characteristics has a particle size of 450 µm or less, which is not large and has a low absorption rate. This mechanism can be considered as follows.

The water absorption behavior of the water-absorbent resin particles is caused by an osmotic pressure generated between the water-absorbent resin particles with a high ion concentration (for example, containing a polyacrylate partially neutralized with sodium) and the liquid to be absorbed with a relatively low ion concentration. Therefore, as an ion concentration in the liquid to be absorbed increases, the osmotic pressure decreases, a water absorption capacity also decreases, and water absorption characteristics such as an absorption amount and an absorption rate weaken. **In** other words, in the water-absorbent resin composition in which the acidic compound is present in the vicinity of the water-absorbent resin particles, an acidic compound in the vicinity of the water-absorbent resin particles is locally dissolved during water absorption, an ion concentration in the vicinity of the surface of the water-absorbent resin particles increases, and the water absorption characteristics, particularly the absorption rate, suitably decrease. Therefore, the absorption rate can decrease without increasing the particle size of the water-absorbent resin composition. Hereinafter, the water-absorbent resin composition of the present invention will be described in detail.

From a viewpoint of more suitably exhibiting an effect of the present invention, the acidic compound is preferably a solid in an environment of a normal temperature (25°C) and a normal pressure (1 atm). From the same viewpoint, the acidic compound is preferably water-soluble. Additionally, in the present invention, the term "water-soluble" means that water has a solubility of 0.5 mass% or more at the normal temperature and the normal pressure.

From the same viewpoint, a first acid dissociation constant of the acidic compound at 25°C is preferably 0.1 to 5.0, more preferably 0.5 to 4.5, and still more preferably 1.0 to 4.0. Furthermore, when the acidic compound has a second acid dissociation constant, the second acid dissociation constant is preferably 2.0 to 7.0, more preferably 2.5 to 6.5, and still more preferably 3.0 to 6.0. In addition, when the acidic compound has a third acid dissociation constant, the third acid dissociation constant is preferably 3.0 to 7.0, more preferably 3.5 to 6.5, and still more preferably 4.0 to 6.0.

From the same viewpoint, the median particle size of the acidic compound is 100 µm or more, or 120 µm or more. On the other hand, the median particle size of the acidic compound is 300 µm or less, or 280 µm or less. In other words, the median particle size of the acidic compound is 100 to 300 µm. The median particle size of the acidic compound can be measured using JIS standard sieves, and is specifically a value measured using a method described in Examples.

From the same viewpoint, the ratio (T/S) of the median particle size T (µm) of the water-absorbent resin particles to the median particle size S (µm) of the acidic compound is preferably 0.1 to 30, more preferably 0.5 to 20, still more preferably 0.8 to 15, and further more preferably 1.0 to 10.

The acidic compound is preferably an organic acid, and among organic acids, tartaric acid, citric acid, malic acid, fumaric acid, sorbic acid, maleic acid, salicylic acid, succinic acid, adipic acid, glutaric acid, glycolic acid, phthalic acid, mandelic acid, and benzoic acid are particularly preferable. Furthermore, tartaric acid, citric acid, malic acid, and fumaric acid are more preferable. The acidic compound contained in the water-absorbent resin composition of the present invention may be one kind or two or more kinds.

From the viewpoint of more suitably exhibiting the effect of the present invention, a lower limit of the content of the acidic compound in the water-absorbent resin composition of the present invention is preferably 0.05 parts by mass or more, more preferably 0.1 parts by mass or more, still more preferably 0.5 parts by mass or more, and further more preferably 1 part by mass relative to 100 parts by mass of the water-absorbent resin particles. In addition, from a viewpoint of ease of industrial production and cost, an upper limit of the content of the acidic compound relative to 100 parts by mass of the water-absorbent resin is preferably 30 parts by mass or less, more preferably 20 parts by mass or less, still more preferably 15 parts by mass or less, and further more preferably 10 parts by mass or less. Lower limit values and an upper limit values thereof can be arbitrarily combined, and the content of the acidic compound relative to 100 parts by mass of the water-absorbent resin is preferably 0.05 to 30 parts by mass, more preferably 0.1 to 20 parts by mass, still more preferably 0.5 to 15 parts by mass, and further more preferably 1 to 10 parts by mass.

From the viewpoint of more suitably exhibiting the effect of the present invention, in the water-absorbent resin composition of the present invention, the difference (B - A) between the absorption rate A (seconds) of the water-absorbent resin particles and the absorption rate B (seconds) of the water-absorbent resin composition is preferably 1 second or more, more preferably 2 seconds or more, still more preferably 3 seconds or more, further more preferably 4 seconds or more, and particularly preferably 5 seconds or more. The difference (B - A) is preferably 70 seconds or less, more preferably 60 seconds or less, still more preferably 50 seconds or less, further more preferably 40 seconds or less, and particularly preferably 30 seconds or less. A range of the difference (B - A) is preferably 1 to 70, more preferably 2 to 60, still more preferably 3 to 50, further more preferably 4 to 40, and particularly preferably 5 to 30.

Then, from the same viewpoint, the absorption rate A (seconds) of the water-absorbent resin particles is 10 seconds or more, preferably 13 seconds or more, and particularly preferably 16 seconds or more. The absorption rate A (seconds) is 40 seconds or less, preferably 35 seconds or less, and particularly preferably 30 seconds or less. A range of the absorption rate A (seconds) is 10 to 40 seconds, preferably 13 to 35 seconds, and particularly preferably 15 to 30 seconds.

Furthermore, from the same viewpoint, the absorption rate B (seconds) of the water-absorbent resin composition is preferably 4 seconds or more, more preferably 7 seconds or more, still more preferably 13 seconds or more, further more preferably 17 seconds or more, and particularly preferably 21 seconds or more. The absorption rate B (seconds) is preferably 130 seconds or less, more preferably 110 seconds or less, still more preferably 90 seconds or less, further more preferably 75 seconds or less, and particularly preferably 60 seconds or less. A range of the absorption rate B (seconds) is preferably 4 to 130 seconds, more preferably 7 to 110 seconds, still more preferably 13 to 90 seconds, further more preferably 17 to 75 seconds, and particularly preferably 21 to 60 seconds.

The absorption rate A (seconds) of the water-absorbent resin particles and the absorption rate B (seconds) of the water-absorbent resin composition are each measured in accordance with the method specified in "Absorption rate test method of water-absorbent resin" in JIS K7224-1996, and specifically, are values measured by the method described in Examples.

Next, the water-absorbent resin particles contained in the water-absorbent resin composition of the present invention will be described in detail.

### (Water-absorbent resin particles)

The water-absorbent resin particles contained in the water-absorbent resin composition of the present invention are formed by crosslinking a polymer of a water-soluble ethylenically unsaturated monomer, that is, a crosslinked polymer with a structural unit derived from a water-soluble ethylenically unsaturated monomer.

Water-absorbent resins are usually in the form of particles. The median particle size of the water-absorbent resin particles is 300 µm or more, or 350 µm or more from a viewpoint of avoiding local absorption in the absorbent article. In addition, the median particle size of the water-absorbent resin particles is 450 µm or less, or 400 µm or less from a viewpoint of creating a comfortable tactile sensation in the absorbent article. In other words, the median particle size is 300 to 450 µm, and preferably 350 to 400 µm. Also in the water-absorbent resin composition of the present invention, the median particle size is 300 to 450 µm, and preferably 350 to 400 µm.

Furthermore, the water-absorbent resin particles may be in a form of fine particles (primary particles) aggregated together (secondary particles) as well as each consisting of a single particle. Examples of shapes of the primary particles include a substantial spherical shape, an indefinite crushed shape, a plate shape, and the like. Examples of the primary particles produced by a reversed-phase suspension polymerization include substantial spherical single particles with a smooth surface shape such as a perfect spherical shape and an elliptical spherical shape, and the primary particles with such a shape have high fluidity as a powder due to the smooth surface shape, and are less likely to be broken specifically when subjected to an impact because the aggregated particles are easily densely packed and become water-absorbent resin particles with high particle strength.

The median particle size of the water-absorbent resin particles can be measured using JIS standard sieves. Specifically, the median particle size is a value measured by a method described in Examples.

As a polymerization method of the water-soluble ethylenically unsaturated monomer, an aqueous solution polymerization method, an emulsion polymerization method, a reversed-phase suspension polymerization method, and the like, which are representative polymerization methods, are used. In the aqueous solution polymerization method, polymerization is performed by heating a water-soluble ethylenically unsaturated monomer solution while stirring the aqueous solution as necessary. Furthermore, in the reversed-phase suspension polymerization method, polymerization is performed by heating the water-soluble ethylenically unsaturated monomer in a hydrocarbon dispersion medium under stirring. The reversed-phase suspension polymerization method is preferably used from a viewpoint of enabling precise polymerization reaction control and wide particle size control.

An example of the method for producing the water-absorbent resin particles will be described below.

Specific examples of the method for producing the water-absorbent resin particles include a method for producing water-absorbent resin particles by subjecting the water-soluble ethylenically unsaturated monomer to the reversed-phase suspension polymerization in the hydrocarbon dispersion medium, the method including steps of: performing polymerization in the presence of a radical polymerization initiator; and post-crosslinking the hydrous gel-like material obtained by polymerization in the presence of a post-crosslinking agent. Furthermore, in the method for producing water-absorbent resin particles of the present invention, an internal crosslinking agent may be added to the water-soluble ethylenically unsaturated monomer as necessary to form the hydrous gel-like material with an internally crosslinked structure.

### <Polymerization step>

### [Water-soluble ethylenically unsaturated monomer]

Examples of the water-soluble ethylenically unsaturated monomer include (meth)acrylic acid (in the present description, "acrylic" and "methacrylic" are collectively referred to as "(meth)acrylic"; and the same applies hereinafter) and salts thereof; 2-(meth)acrylamide-2 methylpropane sulfonic acid and salts thereof; nonionic monomers such as (meth)acrylamide, N,N-dimethyl(meth)acrylamide, 2-hydroxyethyl(meth)acrylate, N-methylol(meth)acrylamide, and polyethylene glycol mono(meth)acrylate; amino group-containing unsaturated monomers such as N,N-diethylaminoethyl(meth)acrylate, N,N-diethylaminopropyl(meth)acrylate, and diethylaminopropyl(meth)acrylamide, and quaternary products thereof; and the like. Among these water-soluble ethylenically unsaturated monomers, the (meth)acrylic acid or salts thereof, (meth)acrylamide, and N,N-dimethylacrylamide are preferable, and the (meth)acrylic acid and salts thereof are more preferable from a viewpoint of industrial availability and the like. In addition, these water-soluble ethylenically unsaturated monomers may be used alone or in a combination of two or more kinds thereof.

Among them, the acrylic acid and salts thereof are widely used as raw materials of water-absorbent resins, and such acrylic acid and/or salts thereof may be copolymerized with the other water-soluble ethylenically unsaturated monomers mentioned above and used. In this case, acrylic acid and/or salts thereof is preferably used as a main water-soluble ethylenically unsaturated monomer in an amount of 70 to 100 mol% with respect to a total amount of water-soluble ethylenically unsaturated monomers.

The water-soluble ethylenically unsaturated monomer is preferably dispersed in the hydrocarbon dispersion medium in a state of an aqueous solution and subjected to the reversed-phase suspension polymerization. When the water-soluble ethylenically unsaturated monomer is an aqueous solution, a dispersion efficiency in the hydrocarbon dispersion medium can be increased. The concentration of the water-soluble ethylenically unsaturated monomer in the aqueous solution is preferably in a range of 20 mass% to a saturated concentration or less. Furthermore, the concentration of the water-soluble ethylenically unsaturated monomer is more preferably 55 mass% or less, still more preferably 50 mass% or less, and still more preferably 45 mass% or less. On the other hand, the concentration of the water-soluble ethylenically unsaturated monomer is more preferably 25 mass% or more, still more preferably 28 mass% or more, and further more preferably 30 mass% or more.

When the water-soluble ethylenically unsaturated monomer has an acid group such as (meth)acrylic acid or 2-(meth)acrylamide-2-methylpropane sulfonic acid, the acid group may be neutralized in advance with an alkaline neutralizing agent as necessary. Examples of such alkaline neutralizing agents include alkali metal salts such as sodium hydroxide, sodium carbonate, sodium hydrogen carbonate, potassium hydroxide, and potassium carbonate; ammonia and the like. In addition, these alkaline neutralizing agents may be used in a form of an aqueous solution in order to simplify neutralization operations. In addition, the alkaline neutralizing agents described above may be used alone, or may be used in a combination of two or more kinds thereof.

A neutralization degree of the water-soluble ethylenically unsaturated monomer by the alkaline neutralizing agents is preferably 10 to 100 mol%, more preferably 30 to 90 mol%, still more preferably 40 to 85 mol%, and further more preferably 50 to 80 mol% as the neutralization degree with respect to all acid groups of the water-soluble ethylenically unsaturated monomer.

### [Radical polymerization initiator]

Examples of radical polymerization initiators added to the polymerization step include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; azo compounds such as 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis{2-[1-(2-hydroxyethyl)-2-imidazolin-2-yl]propane}dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid); and the like. Among the radical polymerization initiators, the potassium persulfate, the ammonium persulfate, the sodium persulfate, and 2,2'-azobis(2-amidinopropane)dihydrochloride are preferable from a viewpoint of easy availability and easy handling. The radical polymerization initiators may be used alone or in combination of two or more. Furthermore, the radical polymerization initiators can also be used as redox polymerization initiators in combination with reducing agents such as sodium sulfite, sodium bisulfite, ferrous sulfate, or L-ascorbic acid.

An amount of the radical polymerization initiators used is, for example, 0.00005 to 0.01 mol relative to 1 mol of the water-soluble ethylenically unsaturated monomer. By satisfying such an amount to be used, an occurrence of a rapid polymerization reaction can be avoided, and the polymerization reaction can be completed within an appropriate time.

### [Internal crosslinking agent]

Examples of internal crosslinking agents include those capable of crosslinking a polymer of the water-soluble ethylenically unsaturated monomer to be used, and for example, (poly)ethylene glycol ["(poly)" in (poly)ethylene glycol means a case where there is or is not a prefix of "poly"; and the same applies hereinafter]; unsaturated polyesters obtained by reacting polyols such as diols and triols like (poly)propylene glycol, 1,4-butanediol, trimethylolpropane, and (poly)glycerin with unsaturated acids such as (meth)acrylic acid, maleic acid, and fumaric acid; bisacrylamides such as N,N-methylenebisacrylamide; di(meth)acrylic acid esters or tri(meth)acrylic acid esters obtained by reacting polyepoxide with (meth)acrylic acid; di(meth)acrylic acid carbamyl esters obtained by reacting polyisocyanate like tolylene diisocyanate and hexamethylene diisocyanate with hydroxyethyl(meth)acrylate; compounds with two or more polymerizable unsaturated groups, such as allylated starch, allylated cellulose, diallyl phthalate, N,N',N"-triallyl isocyanurate, and divinylbenzene; diglycidyl compounds such as (poly)ethylene glycol diglycidyl ether, (poly)propylene glycol diglycidyl ether, and (poly) glycerin diglycidyl ether, and polyglycidyl compounds such as triglycidyl compounds; epihalohydrin compounds such as epichlorohydrin, epibromhydrin, and α-methylepichlorohydrin; compounds with two or more reactive functional groups such as isocyanate compounds like 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; and the like. Among the internal crosslinking agents, unsaturated polyesters or polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are preferably used. The internal crosslinking agents may be used alone, or may be used in a combination of two or more kinds thereof.

An amount of the internal crosslinking agents to be used is preferably 0.000001 to 0.02 mol, more preferably 0.00001 to 0.01 mol, still more preferably 0.00001 to 0.005 mol, and further more preferably 0.00005 to 0.002 mol, relative to 1 mol of the water-soluble ethylenically unsaturated monomer.

### [Hydrocarbon dispersion medium]

Examples of hydrocarbon dispersion mediums include aliphatic hydrocarbons with 6 to 8 carbon atoms such as n-hexane, n-heptane, 2-methylhexane, 3-methylhexane, 2,3-dimethylpentane, 3-ethylpentane, and n-octane; alicyclic hydrocarbon such as cyclohexane, methylcyclohexane, cyclopentane, methylcyclopentane, trans-1,2-dimethylcyclopentane, cis-1,3-dimethylcyclopentane, and trans-1,3-dimethylcyclopentane; aromatic hydrocarbons such as benzene, toluene, and xylene; and the like. Among the hydrocarbon dispersion mediums, n-hexane, n-heptane, and cyclohexane are particularly preferably used because these compounds are industrially easily available, have stable quality, and are inexpensive. The hydrocarbon dispersion mediums may be used alone or in a combination of two or more kinds thereof. In addition, as an example of a mixture of the hydrocarbon dispersion mediums, a suitable result can be obtained by using a commercially available product such as exol heptane (manufactured by Exxon Mobil Corporation: containing 75 to 85 mass% of hydrocarbon of heptane and isomers thereof).

An amount of the hydrocarbon dispersion mediums to be used is preferably 100 to 1500 parts by mass, and more preferably 200 to 1400 parts by mass relative to 100 parts by mass of a first-stage water-soluble ethylenically unsaturated monomer from a viewpoint of uniformly dispersing the water-soluble ethylenically unsaturated monomer and easily controlling a polymerization temperature. **In** addition, as will be described later, the reversed-phase suspension polymerization is performed in one stage (single stage) or two or more stages, and the first-stage polymerization described above means a first-stage polymerization reaction in a single-stage polymerization or a multistage polymerization (the same applies hereinafter).

### [Dispersion stabilizer]

### (Surfactant)

In the reversed-phase suspension polymerization, a dispersion stabilizer can also be used in order to improve a dispersion stability of the water-soluble ethylenically unsaturated monomer in the hydrocarbon dispersion medium. As the dispersion stabilizer, a surfactant can be used.

As surfactants, for example, sucrose fatty acid ester, polyglycerol fatty acid ester, sorbitan fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, a polyoxyethylene polyoxypropylene block copolymer, polyoxyethylene polyoxypropyl alkyl ether, polyethylene glycol fatty acid ester, alkyl glucoside, N-alkyl gluconamide, polyoxyethylene fatty acid amide, polyoxyethylene alkylamine, phosphoric acid ester of polyoxyethylene alkyl ether, phosphoric acid ester of polyoxyethylene alkyl allyl ether, and the like can be used. Among the surfactants, it is particularly preferable to use sorbitan fatty acid ester, polyglycerin fatty acid ester, or sucrose fatty acid ester from a viewpoint of dispersion stability of the monomer. The surfactants may be used alone or in a combination of two or more kinds thereof.

An amount of the surfactant to be used is preferably 0.1 to 30 parts by mass, and more preferably 0.3 to 20 parts by mass, relative to 100 parts by mass of the first-stage water-soluble ethylenically unsaturated monomer.

### (Polymeric dispersant)

As the dispersion stabilizer used in the reversed-phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymer, maleic anhydride-modified EPDM (ethylene-propylene-diene terpolymer), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, ethyl hydroxyethyl cellulose, and the like. Among the polymeric dispersants, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are particularly preferably used from a viewpoint of dispersion stability of monomers. These polymeric dispersants may be used alone or in a combination of two or more kinds thereof.

An amount of the polymeric dispersants to be used is preferably 0.1 to 30 parts by mass, and more preferably 0.3 to 20 parts by mass relative to 100 parts by mass of the first-stage water-soluble ethylenically unsaturated monomer.

### [Other components]

**In** a method for producing water-absorbent resin particles, if desired, other components may be added to an aqueous solution containing the water-soluble ethylenically unsaturated monomer to perform the reversed-phase suspension polymerization. As other components, various additives such as a thickener and a chain transfer agent can be added.

As an example, the reversed-phase suspension polymerization can be performed by adding a thickener to an aqueous solution containing the water-soluble ethylenically unsaturated monomer. By thus adding a thickener to adjust a viscosity of the aqueous solution, it is possible to control a median particle size obtained in the reversed-phase suspension polymerization.

As a thickener, for example, hydroxyethyl cellulose, hydroxypropyl cellulose, methyl cellulose, carboxymethyl cellulose, polyacrylic acid, a (partial) neutralized polyacrylic acid, polyethylene glycol, polyacrylamide, polyethyleneimine, dextrin, sodium alginate, polyvinyl alcohol, polyvinyl pyrrolidone, polyethylene oxide, and the like can be used. In addition, when a stirring rate during polymerization is the same, primary particles and/or secondary particles of the obtained particles tend to be larger as a viscosity of the water-soluble ethylenically unsaturated monomer aqueous solution becomes higher.

### [Reversed-phase suspension polymerization]

In performing the reversed-phase suspension polymerization, for example, an aqueous monomer solution containing the water-soluble ethylenically unsaturated monomer is dispersed in the hydrocarbon dispersion medium in the presence of the dispersion stabilizer. At this time, before a polymerization reaction is started, the addition timing of the dispersion stabilizer (the surfactant or the polymeric dispersant) may be either before or after the addition of the aqueous monomer solution.

Among them, from a viewpoint of easily reducing the amount of the hydrocarbon dispersion medium remaining in the obtained water-absorbent resin particles, it is preferable to disperse the aqueous monomer solution in the hydrocarbon dispersion medium in which the polymeric dispersant is dispersed, and then further disperse the surfactant to perform polymerization.

Such reversed-phase suspension polymerization can be performed in one stage or two or more stages. In addition, from a viewpoint of enhancing productivity, it is preferable to perform in two to three stages.

When the reversed-phase suspension polymerization is performed in two or more stages, after a first-stage reversed-phase suspension polymerization is performed, the water-soluble ethylenically unsaturated monomer may be added to and mixed with the reaction mixture obtained in the first-stage polymerization reaction, and a second-stage or later reversed-phase suspension polymerization may be performed in a same manner as the first-stage polymerization. In the reversed-phase suspension polymerization in each of the second and subsequent stages, in addition to the water-soluble ethylenically unsaturated monomer, the radical polymerization initiator is preferably added within a range of a molar ratio of each component to the water-soluble ethylenically unsaturated monomer described above based on the amount of the water-soluble ethylenically unsaturated monomer added in the reversed-phase suspension polymerization in each of the second and subsequent stages to perform the reversed-phase suspension polymerization. In addition, in the second and subsequent stages of polymerization, an internal crosslinking agent may be added to the water-soluble ethylenically unsaturated monomer as necessary.

A reaction temperature of the polymerization reaction is preferably 20 to 110°C and more preferably 40 to 90°C from a viewpoint of enhancing economic efficiency by rapidly progressing the polymerization and shortening polymerization time, and easily removing heat of polymerization to smoothly perform the reaction.

### <Post-crosslinking step>

Next, water-absorbent resin particles of the present invention are obtained by adding a post-crosslinking agent to a hydrous gel-like material with an internally crosslinked structure obtained by polymerizing the water-soluble ethylenically unsaturated monomer, and crosslinking the hydrous gel-like material (a post-crosslinking reaction). The post-crosslinking reaction is preferably performed in the presence of the post-crosslinking agent after polymerization of the water-soluble ethylenically unsaturated monomer. As described above, by subjecting the hydrous gel-like material with an internally-crosslinked structure to the post-crosslinking reaction after polymerization, it is possible to obtain water-absorbent resin particles in which the crosslinking density in the vicinity of a surface of the water-absorbent resin particles is increased and various performances such as water absorption capacity under load are improved.

Examples of the post-crosslinking agent include compounds with two or more reactive functional groups. For example, polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromhydrin, and α-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylene bisoxazoline; carbonate compounds such as ethylene carbonate; hydroxyalkylamide compounds such as bis[N,N-di(β-hydroxyethyl)]adipamide; and the like. Among the post-crosslinking agent, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferable. The post-crosslinking agent may be used alone, or may be used in a combination of two or more kinds thereof.

An amount of the post-crosslinking agent to be used is preferably 0.00001 to 0.01 mol, more preferably 0.00005 to 0.005 mol, and still more preferably 0.0001 to 0.002 mol, relative to 1 mol of a total amount of the water-soluble ethylenically unsaturated monomers used for polymerization.

As a method for adding the post-crosslinking agent, the post-crosslinking agent may be added as it is or as an aqueous solution, or may be added as a solution using a hydrophilic organic solvent as a solvent as necessary. Examples of the hydrophilic organic solvents include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; sulfoxides such as dimethyl sulfoxide; and the like. The hydrophilic organic solvent may be used alone, or may be used in a combination of two or more kinds thereof, or as a mixed solvent with water.

The addition timing of the post-crosslinking agent may be after almost all the polymerization reaction of the water-soluble ethylenically unsaturated monomer is completed, and the post-crosslinking agent is preferably added in the presence of moisture in a range of 1 to 400 parts by mass, more preferably in the presence of moisture in a range of 5 to 200 parts by mass, still more preferably in the presence of moisture in a range of 10 to 100 parts by mass, and further more preferably in the presence of moisture in a range of 20 to 60 parts by mass relative to 100 parts by mass of the water-soluble ethylenically unsaturated monomer. Furthermore, an amount of moisture means a total amount of moisture contained in a reaction system and moisture used as necessary when the post-crosslinking agent is added.

The reaction temperature in the post-crosslinking reaction is preferably 50 to 250°C, more preferably 60 to 180°C, still more preferably 60 to 140°C, and further more preferably 70 to 120°C. Furthermore, the reaction time of the post-crosslinking reaction is preferably 1 to 300 minutes, and more preferably 5 to 200 minutes.

### <Drying step>

A method may include a drying step of removing water, the hydrocarbon dispersion medium, and the like by distillation by externally applying an energy such as heat after performing the reversed-phase suspension polymerization described above. When dehydration is performed from the hydrous gel after the reversed-phase suspension polymerization, the system in which the hydrous gel is dispersed in the hydrocarbon dispersion medium is heated, so that water and the hydrocarbon dispersion medium are temporarily distilled off to the outside of the system by azeotropic distillation. At this time, when only the distilled hydrocarbon dispersion medium is returned into the system, continuous azeotropic distillation becomes possible. In that case, since a temperature in the system during drying is maintained at an azeotropic temperature with the hydrocarbon dispersion medium or lower, it is preferable from a viewpoint that the resin is hardly deteriorated. Subsequently, water and the hydrocarbon dispersion medium are distilled off to obtain water-absorbent resin particles. Various performances of the water-absorbent resin particles to be obtained can be controlled by controlling the treatment conditions in the drying step after the polymerization to adjust an amount of water to be removed.

In the drying step, drying treatment by distillation may be performed under a normal pressure or under a reduced pressure. In addition, from a viewpoint of enhancing a drying efficiency, the drying may be performed under a flow of nitrogen or the like. When the drying treatment is performed under the normal pressure, a drying temperature is preferably 70 to 250°C, more preferably 80 to 180°C, still more preferably 80 to 140°C, and further more preferably 90 to 130°C. When the drying treatment is performed under the reduced pressure, a drying temperature is preferably 40 to 160°C, and more preferably 50 to 110°C.

When the post-crosslinking step with the post-crosslinking agent is performed after the polymerization of the monomer is performed by reversed-phase suspension polymerization, the drying step by distillation described above is performed after the completion of the post-crosslinking step. Alternatively, the post-crosslinking step and the drying step may be performed simultaneously.

The water-absorbent resin composition of the present invention may contain an additive according to a purpose in addition to the acidic compound. Examples of such additives include inorganic powders, surfactants, oxidants, reducing agents, metal chelating agents, radical chain inhibitors, antioxidants, and antibacterial agents. For example, a fluidity of the water-absorbent resin composition can be further improved by adding 0.05 to 5 parts by mass of amorphous silica as an inorganic powder to 100 parts by mass of the water-absorbent resin particles.

In addition, in the water-absorbent resin composition of the present invention, a content of the water-absorbent resin particles (excluding additives) is preferably 70 mass% or more, more preferably 80 mass% or more, and still more preferably 90 mass% or more.

The water-absorbent resin composition of the present invention can be suitably produced, for example, by a method including a step of mixing water-absorbent resin particles, which are crosslinked polymers of the water-soluble ethylenically unsaturated monomer, the internal crosslinking agent, and the post-crosslinking agent described above, with the acidic compound. A temperature of the mixing step may be 0 to 90°C. A temperature in the mixing step is preferably 15 to 70°C and a relative humidity is preferably 30 to 75%. For example, by mixing the water-absorbent resin particles and the acidic compound in a solid phase state, the acidic compound can be present on a surface of the water-absorbent resin particles to such an extent that an effect of the present invention can be exhibited. In addition, the water-absorbent resin composition of the present invention may be prepared by mixing the acidic compound with water-absorbent resin particles in a state of being dispersed in a liquid medium such as an aqueous liquid.

From the viewpoint of more suitably exerting the effect of the present invention, an addition amount of the acidic compound in the method for producing the water-absorbent resin composition of the present invention is preferably 0.05 to 30 parts by mass, more preferably 0.1 to 20 parts by mass, still more preferably 0.5 to 15 parts by mass, and further more preferably 1 to 10 parts by mass, relative to 100 parts by mass of the water-absorbent resin particles.

From the same viewpoint, the ratio (T/S) of a median particle size T (µm) of the water-absorbent resin particles to a median particle size S (µm) of the acidic compound in the method for producing the water-absorbent resin composition of the present invention is preferably 0.1 to 30, more preferably 0.5 to 20, still more preferably 0.8 to 15, and further more preferably 1.0 to 10.

A method for reducing the absorption rate of water-absorbent resin particles of the present invention can be said to be a method in which an acidic compound with a median particle size of 100 to 300 µm is mixed, preferably attached, to water-absorbent resin particles with a median particle size of 300 to 450 µm.

### 2. Absorber, absorbent article

The water-absorbent resin composition of the present invention constitutes an absorber used for sanitary materials such as sanitary products and disposable diapers, and is suitably used for an absorbent article including the absorber.

Here, the absorber using the water-absorbent resin composition of the present invention contains, as essential constitutional units, water-absorbent resin particles with a median particle size of 300 to 450 µm and an acidic compound with a median particle size of 100 to 300 µm. The absorber may further include hydrophilic fibers. As a form in which the absorber contains the water-absorbent resin particles and the acidic compound, a form in which the water-absorbent resin particles and the acidic compound are adjacent to each other by forming a layer may be adopted, a form in which the water-absorbent resin particles and the hydrophilic fibers are mixed so as to have a uniform composition, and the acidic compound is adjacent to the outside of the mixture may be adopted, or a form in which the water-absorbent resin particles and the acidic compound are sandwiched between a plurality of hydrophilic fiber layers may be adopted. The ratio of the water-absorbent resin particles to the acidic compound in such a form is preferably 0.05 to 30 parts by mass, more preferably 0.1 to 20 parts by mass, still more preferably 0.5 to 15 parts by mass, and further more preferably 1 to 10 parts by mass, relative to 100 parts by mass of the water-absorbent resin particles.

The absorber using the water-absorbent resin composition of the present invention more preferably contains the water-absorbent resin composition of the present invention. The absorber may further include hydrophilic fibers. Examples of configurations of the absorber include a sheet-like structure in which the water-absorbent resin composition is fixed on a nonwoven fabric or between a plurality of nonwoven fabrics, a mixed dispersion obtained by mixing the water-absorbent resin composition and hydrophilic fibers so as to have a uniform composition, a sandwich structure in which the water-absorbent resin composition is sandwiched between layered hydrophilic fibers, a structure in which the water-absorbent resin composition and the hydrophilic fibers are wrapped with tissue, and the like. In addition, the absorber may contain other components, for example, an adhesive binder such as a heat-sealable synthetic fiber, a hot melt adhesive, or an adhesive emulsion for enhancing a shape retention of the absorber.

A content of the water-absorbent resin composition in the absorber is preferably 5 to 100 mass%, more preferably 10 to 95 mass%, still more preferably 20 to 90 mass%, and further more preferably 30 to 80 mass%.

Examples of the hydrophilic fibers include cellulose fibers such as fluff pulp obtained from wood, mechanical pulp, chemical pulp, and semi-chemical pulp; artificial cellulose fibers such as rayon and acetate; and fibers made of synthetic resins such as hydrophilized polyamide, polyester, and polyolefin; and the like. An average fiber length of the hydrophilic fibers is usually 0.1 to 10 mm or may be 0.5 to 5 mm.

The absorber using the water-absorbent resin composition of the present invention can be held between a liquid-permeable sheet (a top sheet) through which a liquid can pass and a liquid-impermeable sheet (a back sheet) through which a liquid cannot pass to form the absorbent article of the present invention. The liquid-permeable sheet is disposed on a side in contact with the body, and the liquid-impermeable sheet is disposed on the opposite side in contact with the body.

Examples of the liquid permeable sheet include nonwoven fabrics such as an air-through type, a spunbond type, a chemical bond type, and a needle punch type made of fibers such as polyethylene, polypropylene, and polyester, porous synthetic resin sheets, and the like. Examples of the liquid-impermeable sheet include synthetic resin films made of resins such as polyethylene, polypropylene, and polyvinyl chloride, and the like.

### EXAMPLES

Hereinafter, the present invention will be described in detail with reference to the examples and comparative examples. However, the present invention is not limited to the examples.

The water-absorbent resin compositions obtained in the following examples and comparative examples were evaluated in various tests below. In addition, unless otherwise specified, the measurement was performed under an environment of a temperature of 25 ± 2°C and a humidity of 50 ± 10%. Hereinafter, each evaluation test method will be described.

### <Absorption rate>

Absorption rates of each of the water-absorbent resin compositions and the water-absorbent resin particles were measured according to the following procedure based on a vortex method (JIS K7224-1996). To start with, 0.05 parts by mass of Blue No. 1 was mixed with 2000 parts by mass of ion-exchanged water to prepare colored ion-exchanged water, and a temperature was adjusted to 25 ± 0.2°C in a constant temperature water bath. The colored ion-exchanged water (50 ± 0.01 g) was weighed in a beaker with a volume of 100 mL. Next, a stirrer (8 mmφ × 30 mm, no ring) was put in a beaker and stirred at a rotation speed of 600 rpm using a magnetic stirrer to generate a vortex. The time (seconds) from when 0.5 ± 0.0002 g of the water-absorbent resin composition was put into the beaker until the stirrer was covered with the gelled ion-exchanged water was measured. The measurement was performed five times, and average values thereof were defined as the absorption rate B of the water-absorbent resin composition and the absorption rate A of the water-absorbent resin particles, respectively. A difference (B - A) between the absorption rate A of the water-absorbent resin particles and the absorption rate B of the water-absorbent resin composition is shown in Table 1.

### <Median particle size of water-absorbent resin particles>

JIS standard sieves were combined in the following order from the top: a sieve with a mesh size of 850 µm, a sieve with a mesh size of 600 µm, a sieve with a mesh size of 500 µm, a sieve with a mesh size of 425 m, a sieve with a mesh size of 300 µm, a sieve with a mesh size of 250 µm, a sieve with a mesh size of 150 µm, and a receptacle. Water-absorbent resin particles (50 g) were placed on the uppermost sieve of combined sieves, and shaken for 10 minutes using a Ro-Tap type (Rotating and Tapping type) shaker to perform classification. After classification, a mass of the water-absorbent resin particles remaining on each sieve was calculated as a mass percentage with respect to a total amount, and a particle size distribution was determined. With respect to the particle size distribution, a relationship between a mesh size of the sieve and an integrated value of the mass percentage of water-absorbent resin particles remaining on the sieve was plotted on a logarithmic probability paper by integrating the particles on the sieve in a descending order of particle size. By connecting the plots on the probability paper with a straight line, a particle size corresponding to an integrated mass percentage of 50 mass% was defined as a median particle size of the water-absorbent resin particles.

### <Median particle size of acidic compound>

An acidic compound (10 g) was sieved using a continuous fully automatic sonic vibration type sieving measuring instrument (Robot shifter RPS-205, manufactured by Seishin Enterprise Co., Ltd.), a sieve with JIS standard mesh sizes of 850 µm, 500 µm, 425 µm, 300 µm, 212 µm, 106 µm, 75 µm, and 45 µm, and a pan under sieving conditions of a frequency of 80 Hz, a pulse interval of 1 second, and a classification time of 2 minutes. A mass of the acidic compound remaining on each sieve was calculated as a mass percentage with respect to a total amount. A mass percentage of the acidic compound remaining on each sieve was integrated in a descending order of particle size, and a relationship between a mesh size of the sieve and an integrated value of the mass percentage of the acidic compound remaining on the sieve was plotted on a logarithmic probability paper. By connecting plots on the probability paper with a straight line, a particle size corresponding to an integrated mass percentage of 50 mass% was determined, and this was taken as a median particle size of the acidic compound.

### <Physiological saline absorption amount>

In a plastic beaker of 500 mL, physiological saline of 500 g and a stirrer (8 mmφ × 30 mm without ring) were put, and stirred at 600 rpm using a magnetic stirrer. Water-absorbent resin particles (2.0 g) were dispersed in the beaker, and sufficiently swollen by gentle stirring at 600 rpm for 1 hour. On the other hand, a mass Wa (g) of a standard sieve with a mesh size of 75 µm was measured, and an aqueous solution containing a swollen gel was filtered through a standard sieve with a mesh size of 75 µm. A standard sieve of 75 µm was allowed to stand for 30 minutes in a state where an angle formed with respect to the horizontal was inclined to be about 30 degrees, and excess physiological saline was removed from the water-absorbent resin particles. A sieve mass Wb (g) containing the swollen gel was measured, and a mass obtained by subtracting the mass Wa (g) of a 75 µm standard sieve from the mass Wb (g) was divided by the mass (2.0 g) of water-absorbent resin particles to calculate the absorption amount.
$Absorption amount = \left(Wb - Wa\right) \times \left(mass of water-absorbent resin particles\right)$

### <Production Example 1>

A round-bottomed cylindrical separable flask of 2 L having an inner diameter of 11 cm and equipped with a reflux condenser, a dropping funnel, a nitrogen gas introduction tube, and a stirring blade having two stages of 4-inclined paddle blades with a blade diameter of 5 cm was prepared as a stirrer. To this flask, n-heptane as a hydrocarbon dispersion medium of 293 g and maleic anhydride-modified ethylene-propylene copolymer of 0.736 g (Mitsui Chemicals, Inc., Hi-wax 1105 A) as a polymeric dispersant were added, and a temperature was raised to 80°C with stirring to dissolve the dispersant, then a mixture was cooled to 50°C. On the other hand, an 80.5 mass% aqueous solution of acrylic acid of 92.0 g (1.03 mol) as the water-soluble ethylenically unsaturated monomer was placed in a beaker with an internal volume of 300 mL, and a 20.9 mass% aqueous solution of sodium hydroxide of 147.7 g was added dropwise while cooling with ice water from an outside to perform neutralization at 75 mol%. Then, hydroxyl ethyl cellulose of 0.092 g (Sumitomo Seika Chemicals Company, Limited, HECAW -15 F) as a thickener, the potassium persulfate of 0.0736 g (0.272 mmol) as a water-soluble radical polymerization agent, and ethylene glycol diglycidyl ether of 0.010 g (0.057 mmol) as an internal crosslinking agent were added thereto and dissolved, thereby preparing a first-stage aqueous solution. Then, the aqueous solution prepared above was added to a separable flask and stirred for 10 minutes, and then a surfactant solution obtained by heating and dissolving sucrose stearate ester of 0.736 g (Ryoto sugar ester S -370 manufactured by Mitsubishi Chemical Foods Corporation) with HLB3 as a surfactant in n-heptane of 6.62 g was further added. While a rotation speed of a stirrer was set to 550 rpm and stirring, an inside of the system was sufficiently replaced with nitrogen. Thereafter, the flask was immersed in a water bath at 70°C and heated, and polymerization was performed for 60 minutes to obtain a first-stage polymerization slurry solution. On the other hand, an 80.5 mass% aqueous solution of acrylic acid of 128.8 g (1.43 mol) as the water-soluble ethylenically unsaturated monomer was placed in another beaker with an internal volume of 500 mL, a 27 mass% aqueous solution of sodium hydroxide of 159.0 g was added dropwise with external cooling to perform neutralization at 75 mol%, and then the potassium persulfate of 0.103 g (0.381 mmol) as a water-soluble radical polymerization initiator and ethylene glycol diglycidyl ether of 0.0116 g (0.067 mmol) as an internal crosslinking agent were added and dissolved, thereby preparing a second-stage aqueous liquid. An inside of a separable flask system was cooled to 25°C while a rotation speed of the stirrer was set to 1000 rpm, and then a whole amount of a second-stage aqueous liquid was added to the first-stage polymerization slurry, and the inside of the system was replaced with nitrogen for 30 minutes. Then, the flask was immersed in a water bath at 70°C again, the temperature was raised, and the polymerization reaction was performed for 60 minutes to obtain a hydrous gel polymer. A 45 mass% aqueous pentasodium diethylenetriamine pentaacetate solution (0.589 g) was added to a hydrous gel polymer after a second-stage polymerization under stirring. Thereafter, the flask was immersed in an oil bath set at 125°C, and water of 257.7 g was removed to the outside of the system while refluxing n-heptane by azeotropic distillation of n-heptane and water. Thereafter, a 2 mass% aqueous solution of ethylene glycol diglycidyl ether of 4.42 g (0.507 mmol) as a surface crosslinking agent was added to the flask, and the mixture was held at 83°C for 2 hours. Thereafter, n-heptane was evaporated at 125°C and dried to obtain polymer particles (dry product). The polymer particles were passed through a sieve with a mesh size of 850 µm to obtain 228.0 g of water-absorbent resin particles. A median particle size of the water-absorbent resin particles was 394 µm, and a physiological saline absorption amount was 61 g/g.

### <Example 1>

L-tartaric acid (0.5 parts by mass) (manufactured by FUSO CHEMICAL INDUSTRY CO., LTD., product name: purified L-tartaric acid, a first acid dissociation constant pKa1 = 2.87, a second acid dissociation constant pKa2 = 3.97, a median particle size 280 µm) was added to 100 parts by mass of the water-absorbent resin particles obtained in Production Example 1, and was mixed for 30 minutes (conditions: a revolution speed 50 rpm, a rotation speed 50 rpm) under environment of a temperature of 25°C and a relative humidity of 50% using a cross rotary mixer manufactured by Meiwa Industries, Ltd., to obtain a water-absorbent resin composition.

### <Example 2>

A water-absorbent resin composition was obtained in the same manner as in Example 1 except that L-tartaric acid was changed to 1.0 parts by mass relative to 100 parts by mass of the water-absorbent resin particles in Example 1.

### <Example 3>

A water-absorbent resin composition was obtained in the same manner as in Example 1 except that L-tartaric acid was changed to 2.0 parts by mass relative to 100 parts by mass of the water-absorbent resin particles in Example 1.

### <Example 4>

A water-absorbent resin composition was obtained in the same manner as in Example 1 except that L-tartaric acid was changed to 3.0 parts by mass relative to 100 parts by mass of the water-absorbent resin particles in Example 1.

### <Example 5>

A water-absorbent resin composition was obtained in the same manner as in Example 1 except that L-tartaric acid was changed to 5.0 parts by mass relative to 100 parts by mass of the water-absorbent resin particles in Example 1.

### <Example 6>

A water-absorbent resin composition was obtained in the same manner as in Example 1 except that L-tartaric acid was changed to 10.0 parts by mass relative to 100 parts by mass of the water-absorbent resin particles in Example 1.

### <Example 7>

Citric acid (1.0 part by mass) (manufactured by FUSO CHEMICAL INDUSTRY CO., LTD., product name: Fuso citrate (anhydrous), a first acid dissociation constant pKa1 = 2.90, a second acid dissociation constant pKa2 = 4.35, a third acid dissociation constant pKa3 = 5.69, a median particle size 236 µm) was added to 100 parts by mass of the water-absorbent resin particles obtained in Production Example 1, and the mixture was mixed under environment of a temperature of 25°C and a relative humidity of 50% for 30 minutes (conditions: a revolution speed 50 rpm, a rotation speed 50 rpm) using a cross rotary mixer manufactured by Meiwa Industries, Ltd., to obtain a water-absorbent resin composition.

### <Example 8>

DL-malic acid (1.0 part by mass) (manufactured by FUSO CHEMICAL INDUSTRY CO., LTD., product name: Fuso malate, a first acid dissociation constant pKa1 = 3.23, a second acid dissociation constant pKa2 = 4.77, a median particle size 156 µm) was added to 100 parts by mass of the water-absorbent resin particles obtained in Production Example 1, and mixed for 30 minutes (conditions: a revolution speed 50 rpm, a rotation speed 50 rpm) under environment of a temperature of 25°C and a relative humidity of 50% using a cross rotary mixer manufactured by Meiwa Industries, Ltd., to obtain a water-absorbent resin composition.

### <Example 9>

Fumaric acid (1.0 part by mass) (manufactured by FUSO CHEMICAL INDUSTRY CO., LTD., product name: fumaric acid, a first acid dissociation constant pKa1 = 3.07, a second acid dissociation constant pKa2 = 4.58, a median particle size 161 µm) was added to 100 parts by mass of the water-absorbent resin particles obtained in Production Example 1, and mixed for 30 minutes (conditions: a revolution speed 50 rpm, a rotation speed 50 rpm) under environment of a temperature of 25°C and a relative humidity of 50% using a cross rotary mixer manufactured by Meiwa Industries, Ltd., to obtain a water-absorbent resin composition.

### <Comparative Example 1>

The water-absorbent resin particles obtained in Production Example 1 were used as they were as water-absorbent resin particles of Comparative Example 1.

**[Table 1]**

| | Water-absorbent resin composition | | | | | | Difference between absorption rates |
|---|---|---|---|---|---|---|---|
| | Acidic compound | | | Water-absorbent resin particles | | Absorption rate B (seconds) | |
| | Type | Addition amount (parts by mass) | Median particle size (µm) | Median particle size (µm) | Absorption rate A (seconds) | | |
| | | | | | | | (B - A) (seconds) |
| Example 1 | L-tartaric acid | 0.5 | 280 | 394 | 29 | 31 | 2 |
| Example 2 | L-tartaric acid | 1 | 280 | 394 | 29 | 33 | 4 |
| Example 3 | L-tartaric acid | 2 | 280 | 394 | 29 | 36 | 7 |
| Example 4 | L-tartaric acid | 3 | 280 | 394 | 29 | 35 | 6 |
| Example 5 | L-tartaric acid | 5 | 280 | 394 | 29 | 44 | 15 |
| Example 6 | L-tartaric acid | 10 | 280 | 394 | 29 | 56 | 27 |
| Example 7 | Citric acid | 1 | 236 | 394 | 29 | 33 | 4 |
| Example 8 | DL-malic acid | 1 | 156 | 394 | 29 | 33 | 4 |
| Example 9 | Fumaric acid | 1 | 161 | 394 | 29 | 30 | 1 |
| Comparative Example 1 | - | | - | 394 | 29 | - | - |

## Claims

1. A water-absorbent resin composition comprising:
water-absorbent resin particles with a median particle size, which is determined by the method described in the description, of 300 to 450 µm; and
an acidic compound with a median particle size, which is determined by the method described in the description, of 100 to 300 µm,
wherein an absorption rate A of the water-absorbent resin particles, which is determined by the method described in the description, is 10 to 40 seconds.

2. The water-absorbent resin composition according to claim 1, wherein a difference (B - A) between an absorption rate A of the water-absorbent resin particles and an absorption rate B of the water-absorbent resin composition is 1 second or more, wherein the absorption rates A and B are determined by the method described in the description.

3. The water-absorbent resin composition according to claim 1 or 2, wherein the absorption rate B of the water-absorbent resin composition is 4 to 130 seconds, wherein the absorption rate B is determined by the method described in the description.

4. The water-absorbent resin composition according to any one of claims 1 to 3, wherein a first acid dissociation constant of the acidic compound is 0.1 to 5.0.

5. An absorber comprising:
water-absorbent resin particles with a median particle size, which is determined by the method described in the description, of 300 to 450 µm; and
an acidic compound with a median particle size, which is determined by the method described in the description, of 100 to 300 µm,
wherein an absorption rate A of the water-absorbent resin particles, which is determined by the method described in the description, is 10 to 40 seconds, and
wherein the water-absorbent resin particles and the acidic compound are mixed.

6. An absorbent article comprising the absorber according to claim 5.

7. A method for producing a water-absorbent resin composition, the method comprising a step of mixing water-absorbent resin particles with a median particle size, which is determined by the method described in the description, of 300 to 450 µm and an acidic compound with a median particle size, which is determined by the method described in the description, of 100 to 300 µm, wherein an absorption rate A of the water-absorbent resin particles, which is determined by the method described in the description, is 10 to 40 seconds.

8. The method for producing the water-absorbent resin composition according to claim 7, wherein a temperature in the mixing step is 0 to 90°C and a relative humidity is 30 to 75%.

9. The method for producing the water-absorbent resin composition according to claim 7 or 8, wherein an amount of the acidic compound is 0.05 to 30 parts by mass relative to 100 parts by mass of the water-absorbent resin particles.

10. The method for producing the water-absorbent resin composition according to any one of claims 7 to 9, wherein a ratio (T/S) of a median particle size T (µm) of the water-absorbent resin particles to a median particle size S (µm) of the acidic compound is 0.1 to 30.

11. A method for reducing an absorption rate of water-absorbent resin particles, the method comprising a step of mixing an acidic compound with a median particle size, which is determined by the method described in the description, of 100 to 300 µm and water-absorbent resin particles with a median particle size, which is determined by the method described in the description, of 300 to 450 µm, wherein an absorption rate A of the water-absorbent resin particles, which is determined by the method described in the description, is 10 to 40 seconds.

## Patentansprüche

1. Wasserabsorbierende Harzzusammensetzung, die Folgendes umfasst:
wasserabsorbierende Harzteilchen mit einer mittleren Teilchengröße, die durch das in der Beschreibung beschriebene Verfahren bestimmt wird, von 300 bis 450 µm und
eine saure Verbindung mit einer mittleren Teilchengröße, die durch das in der Beschreibung beschriebene Verfahren bestimmt wird, von 100 bis 300 µm;
wobei die Absorptionsrate A der wasserabsorbierenden Harzteilchen, die durch das in der Beschreibung beschriebene Verfahren bestimmt wird, 10 bis 40 s beträgt.

2. Wasserabsorbierende Harzzusammensetzung nach Anspruch 1, wobei die Differenz (B - A) zwischen der Absorptionsrate A der wasserabsorbierenden Harzteilen und der Absorptionsrate B der wasserabsorbierenden Harzzusammensetzung 1 s oder mehr beträgt, wobei die Absorptionsraten A und B durch das in der Beschreibung beschriebene Verfahren bestimmt werden.

3. Wasserabsorbierende Harzzusammensetzung nach Anspruch 1 oder 2, wobei die Absorptionsrate B der wasserabsorbierenden Harzzusammensetzung 4 bis 130 beträgt, wobei die Absorptionsrate B durch das in der Beschreibung beschriebene Verfahren bestimmt wird.

4. Wasserabsorbierende Harzzusammensetzung nach einem der Ansprüche 1 bis 3, wobei eine erste Säuredissoziationskonstante der sauren Verbindung 0,1 bis 5,0 beträgt.

5. Absorptionsmittel, das Folgendes umfasst:
wasserabsorbierende Harzteilchen mit einer mittleren Teilchengröße, die durch das in der Beschreibung beschriebene Verfahren bestimmt wird, von 300 bis 450 µm und
eine saure Verbindung mit einer mittleren Teilchengröße, die durch das in der Beschreibung beschriebene Verfahren bestimmt wird, von 100 bis 300 µm;
wobei die Absorptionsrate A der wasserabsorbierenden Teilchen, die durch das in der Beschreibung beschriebene Verfahren bestimmt wird, 10 bis 40 s beträgt und
worin die wasserabsorbierenden Harzteilchen und die saure Verbindung vermischt sind.

6. Absorbierender Gegenstand, der ein Absorptionsmittel nach Anspruch 5 umfasst.

7. Verfahren zur Herstellung einer wasserabsorbierenden Harzzusammensetzung, wobei das Verfahren einen Schritt des Vermischens von wasserabsorbierenden Harzteilchen mit einer mittleren Teilchengröße, die durch das in der Beschreibung beschriebene Verfahren bestimmt wird, von 300 bis 450 µm und einer sauren Verbindung mit einer mittleren Teilchengröße, die durch das in der Beschreibung beschriebene Verfahren bestimmt wird, von 100 bis 300 µm umfasst, wobei die Absorptionsrate A der wasserabsorbierenden Harzteilchen, die durch das in der Beschreibung beschriebene Verfahren bestimmt wird, 10 bis 40 s beträgt.

8. Verfahren zur Herstellung einer wasserabsorbierenden Harzzusammensetzung nach Anspruch 7, wobei in dem Mischschritt die Temperatur 0 °C bis 90 °C beträgt und die relative Feuchtigkeit 30 % bis 75 % beträgt.

9. Verfahren zur Herstellung einer wasserabsorbierenden Harzzusammensetzung nach Anspruch 7 oder 8, wobei die Menge der sauren Verbindung 0,05 bis 30 Massenteile, bezogen auf 100 Massenteile der wasserabsorbierenden Harzteilchen beträgt.

10. Verfahren zur Herstellung einer wasserabsorbierenden Harzzusammensetzung nach einem der Ansprüche 7 bis 9, wobei das Verhältnis (T/S) zwischen der mittleren Teilchengröße T (µm) der wasserabsorbierenden Harzteilchen und der mittleren Teilchengröße S (µm) der sauren Verbindung 0,1 bis 30 beträgt.

11. Verfahren zum Verringern der Absorptionsrate von wasserabsorbierenden Harzteilchen, wobei das Verfahren einen Schritt des Vermischens einer sauren Verbindung mit einer mittleren Teilchengröße, die durch das in der Beschreibung beschriebene Verfahren bestimmt wird, von 100 bis 300 µm und von wasserabsorbierenden Harzteilchen mit einer mittleren Teilchengröße, die durch das in der Beschreibung beschriebene Verfahren bestimmt wird, von 300 bis 450 µm umfasst, wobei die Absorptionsrate A der wasserabsorbierenden Harzteilchen, die durch das in der Beschreibung beschriebene Verfahren bestimmt wird, 10 bis 40 s beträgt.

## Revendications

1. Composition de résine absorbant l'eau, comprenant :
des particules de résine absorbant l'eau avec une taille de particule médiane, qui est déterminée par le procédé décrit dans la description, de 300 à 450 µm ; et
un composé acide avec une taille de particule médiane, qui est déterminée par le procédé décrit dans la description, de 100 à 300 µm,
dans laquelle une vitesse d'absorption A des particules de résine absorbant l'eau, qui est déterminée par le procédé décrit dans la description, est de 10 à 40 secondes.

2. Composition de résine absorbant l'eau selon la revendication 1, dans laquelle une différence (B - A) entre une vitesse d'absorption A des particules de résine absorbant l'eau et une vitesse d'absorption B de la composition de résine absorbant l'eau est de 1 seconde ou plus, dans laquelle les vitesse d'absorption A et B sont déterminées par le procédé décrit dans la description.

3. Composition de résine absorbant l'eau selon la revendication 1 ou 2, dans laquelle la vitesse d'absorption B de la composition de résine absorbant l'eau est de 4 à 130 secondes, dans laquelle le taux d'absorption B est déterminé par le procédé décrit dans la description.

4. Composition de résine absorbant l'eau selon l'une quelconque des revendications 1 à 3, dans laquelle une première constante de dissociation acide du composé acide est de 0,1 à 5,0.

5. Absorbeur, comprenant :
des particules de résine absorbant l'eau avec une taille de particule médiane, qui est déterminée par le procédé décrit dans la description, de 300 à 450 µm ; et
un composé acide avec une taille de particule médiane, qui est déterminée par le procédé décrit dans la description, de 100 à 300 µm,
dans lequel une vitesse d'absorption A des particules de résine absorbant l'eau, qui est déterminée par le procédé décrit dans la description, est de 10 à 40 secondes, et
dans lequel les particules de résine absorbant l'eau et le composé acide sont mélangés.

6. Article absorbant comprenant l'absorbeur selon la revendication 5.

7. Procédé de production d'une composition de résine absorbant l'eau, le procédé comprenant une étape de mélange de particules de résine absorbant l'eau avec une taille de particule médiane, qui est déterminée par le procédé décrit dans la description, de 300 à 450 µm et d'un composé acide avec une taille de particule médiane, qui est déterminée par le procédé décrit dans la description, de 100 à 300 µm, dans lequel une vitesse d'absorption A des particules de résine absorbant l'eau, qui est déterminée par le procédé décrit dans la description, est de 10 à 40 secondes.

8. Procédé de production de la composition de résine absorbant l'eau selon la revendication 7, dans lequel une température dans l'étape de mélange est de 0 à 90°C et une humidité relative est de 30 à 75 %.

9. Procédé de production de la composition de résine absorbant l'eau selon la revendication 7 ou 8, dans lequel une quantité du composé acide est de 0,05 à 30 parties en masse par rapport à 100 parties en masse des particules de résine absorbant l'eau.

10. Procédé de production de la composition de résine absorbant l'eau selon l'une quelconque des revendications 7 à 9, dans lequel un rapport (T/S) d'une taille de particule médiane T (µm) des particules de résine absorbant l'eau à une taille de particule médiane S (µm) du composé acide est de 0,1 à 30.

11. Procédé de réduction d'une vitesse d'absorption de particules de résine absorbant l'eau, le procédé comprenant une étape de mélange d'un composé acide avec une taille de particule médiane, qui est déterminée par le procédé décrit dans la description, de 100 à 300 µm et de particules de résine absorbant l'eau avec une taille de particule médiane, qui est déterminée par le procédé décrit dans la description, de 300 à 450 µm, dans lequel une vitesse d'absorption A des particules de résine absorbant l'eau, qui est déterminée par le procédé décrit dans la description, est de 10 à 40 secondes.
